(19) **Europäisches Patentamt / European Patent Office / Office européen des brevets**

(11) **EP 3 575 407 B1**

(12) **EUROPEAN PATENT SPECIFICATION**

(45) Date of publication and mention
of the grant of the patent:
**27.03.2024 Bulletin 2024/13**

(21) Application number: **18743980.7**

(22) Date of filing: **10.01.2018**

(51) International Patent Classification (IPC):
***C12Q 1/6869*** (2018.01)      ***C12Q 1/68*** (2018.01)
***C12M 1/00*** (2006.01)

(52) Cooperative Patent Classification (CPC):
(C-Sets available)
**C12Q 1/6869; C12M 1/00; C12Q 1/68**      (Cont.)

(86) International application number:
**PCT/CN2018/072045**

(87) International publication number:
**WO 2018/137496 (02.08.2018 Gazette 2018/31)**

(54) **METHOD FOR DETERMINING PROPORTION OF CELL-FREE NUCLEIC ACIDS FROM PREDETERMINED SOURCE IN BIOLOGICAL SAMPLE**

VERFAHREN ZUR BESTIMMUNG DES ANTEILS AN ZELLFREIEM NUKLEINSÄUREN AUS EINER VORBESTIMMTEN QUELLE IN EINER BIOLOGISCHEN PROBE

PROCÉDÉ POUR DÉTERMINER UNE PROPORTION D'ACIDE NUCLÉOTIDE ACELLULAIRE PROVENANT D'UNE SOURCE PRÉDÉTERMINÉE DANS UN ÉCHANTILLON BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2017 CN 201710055200**

(43) Date of publication of application:
**04.12.2019 Bulletin 2019/49**

(73) Proprietor: **BGI Genomics Co., Ltd.**
**Yantian District**
**Shenzhen, Guangdong Province 518083 (CN)**

(72) Inventors:
• **YUAN, Yuying**
  **Shenzhen**
  **Guangdong 518083 (CN)**
• **CHAI, Xianghua**
  **Shenzhen**
  **Guangdong 518083 (CN)**
• **WANG, Shuyuan**
  **Shenzhen**
  **Guangdong 518083 (CN)**
• **CHEN, Lina**
  **Shenzhen**
  **Guangdong 518083 (CN)**

• **ZHOU, Lijun**
  **Shenzhen**
  **Guangdong 518083 (CN)**
• **LIU, Qiang**
  **Shenzhen**
  **Guangdong 518083 (CN)**
• **ZHANG, Hongyun**
  **Shenzhen**
  **Guangdong 518083 (CN)**
• **WANG, Wei**
  **Shenzhen**
  **Guangdong 518083 (CN)**
• **LIU, Na**
  **Shenzhen**
  **Guangdong 518083 (CN)**
• **YIN, Ye**
  **Shenzhen**
  **Guangdong 518083 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(56) References cited:
**WO-A1-2014/043763    CA-A1- 2 956 105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

CN-A- 105 296 606      CN-A- 105 331 606

- H. C. FAN ET AL: "Analysis of the Size Distributions of Fetal and Maternal Cell-Free DNA by Paired-End Sequencing", CLINICAL CHEMISTRY, vol. 56, no. 8, 1 August 2010 (2010-08-01) , pages 1279-1286, XP055026439, ISSN: 0009-9147, DOI: 10.1373/clinchem.2010.144188
- S. C. Y. YU ET AL: "Size-based molecular diagnostics using plasma DNA for noninvasive prenatal testing", PROCEEDINGS OF THE NATIONAL ACADEMY OF SCIENCES, vol. 111, no. 23, 19 May 2014 (2014-05-19) , pages 8583-8588, XP055297276, US ISSN: 0027-8424, DOI: 10.1073/pnas.1406103111

(52) Cooperative Patent Classification (CPC): (Cont.)

C-Sets
C12Q 1/6869, C12Q 2537/165, C12Q 2545/101, C12Q 2545/114

**Description**

**FIELD**

The present disclosure relates to the field of biotechnology, especially to non-invasive prenatal genetic testing and oncogene testing, and more specifically to a method for determining a fraction of cell-free nucleic acids from a predetermined source in a biological sample.

**BACKGROUND**

Since 1977, researchers have successively found cancer-derived DNA in peripheral blood of patients with tumor, and also confirmed the presence of cell-free fetal (cff)-DNAs in plasma from a pregnant woman. Detection or estimation of a fraction of cancer-derived DNAs in the peripheral blood of patients with tumor or that of cell-free fetal DNAs in the plasma from the pregnant woman, i.e. determination of a fraction of cell-free nucleic acids from a predetermined source in a biological sample, is of great significance.

However, the current method for determining a fraction of cell-free nucleic acids from a predetermined source in a biological sample remains to be improved.

**SUMMARY**

Embodiments of the present disclosure seek to solve at least one of the problems existing in the related art to at least some extent. For this, an object of the present disclosure is to provide a method capable of accurately and efficiently determining a fraction of cell-free nucleic acids from a predetermined source in a biological sample.

It should be noted that the technical solutions of the present disclosure are achieved through the following discoveries.

At present, the fraction of cell-free fetal DNAs in peripheral blood is estimated mainly by the following directions: 1) different methylation level for a particular biomarker between maternal DNA fragments and cell-free fetal DNA fragments in mononuclear cells from peripheral blood; 2) selection of a plurality of representative single nucleotide polymorphism (SNPs) sites each of which is different between maternal DNA fragments and cell-free fetal DNA fragments; 3) different size between fetal DNA fragments and maternal DNA fragments in maternal blood circulation; 4) estimation of the fetal DNA fraction for a pregnant woman with a male fetus by the Y chromosome method. However, these four directions all have individual limitations. In specific, the first one is in high demand on a large amount of plasma; the second one requires probe capture and high sequencing depth, or paternal genetic information; the third one needs to determine the lengths for both the fetal DNA fragments and maternal DNA fragments, resulting in high-cost for sequencing and ordinary accuracy; and the last one is applicable to estimate the fetal DNA fraction only for the pregnant woman with the male fetus, but invalid for a pregnant woman with a female fetus.

In order to overcome the above limitations of these methods, the inventors have developed a method for estimating the fetal DNA fraction which only utilizes sequencing data currently detected by Non-Invasive Prenatal Testing (NIPT), without additional sequencing data. That is, this method is capable of accurately quantifying the fetal DNA fraction in the peripheral blood by low coverage sequencing data. The research and development of this method is mainly based on the discovery that the number of reads in each of windows, obtained by sequential division of an autosome by a certain length, is correlated with the fetal DNA fraction. Utilizing such a discovery, the fetal DNA fraction can be estimated in a high accuracy for not only the male fetus but also the female fetus.

The inventors have found through further researches that this method has wide applications and is applicable to detect free DNAs from different sources. For example, the method is suitable to determine the fraction of tumor-derived cell-free nucleic acids or non-tumor derived cell-free nucleic acids in a peripheral blood sample obtained from a subject suffering from tumor, suspected to suffer from tumor or subjected to tumor screening, and it is also possible to obtain accurate and reliable results by low coverage sequencing data.

Therefore, according to embodiments of an aspect of the present disclosure, there is provided a method for determining a fraction of cell-free nucleic acids from a predetermined source in a biological sample. In embodiments of the present disclosure, the method includes: (1) performing sequencing on cell-free nucleic acids contained in the biological sample, so as to obtain a sequencing result consisting of a plurality of sequencing reads; (2) aligning the sequencing result to a reference sequence, so as to determine the number of sequencing reads falling into a predetermined window in the sequencing result; and (3) determining the fraction of the cell-free nucleic acids from the predetermined source in the biological sample based on the number of the sequencing reads falling into the predetermined window.

The inventors have surprisingly found that, the fraction of the cell-free nucleic acids from the predetermined source in the biological sample, especially the fraction of the cell-free fetal nucleic acids in a peripheral blood sample obtained from a pregnant woman, and the fraction of tumor-derived cell-free nucleic acids in a peripheral blood sample obtained from a subject suffering from tumor, suspected to suffer from tumor or subjected to tumor screening can be accurately and efficiently determined by the method of the present disclosure.

Also disclosed, but not part of the invention, is a device for determining a fraction of cell-free nucleic acids from a predetermined source in a biological sample. The device includes: a sequencing apparatus, configured to perform sequencing on cell-free nucleic acids contained in the biological sample, so as to obtain a sequencing result consisting of a plurality of sequencing reads; a counting apparatus, connected to the sequencing apparatus and configured to align the sequencing result to a reference sequence, so as to determine the number of sequencing reads falling into a predetermined window in the sequencing result; and a fraction of cell-free nucleic acids determining apparatus, connected to the counting apparatus and configured to determine the fraction of the cell-free nucleic acids from the predetermined source in the biological sample based on the number of the sequencing reads falling into the predetermined window.

The device of the present disclosure is suitable to carry out the method for determining a fraction of cell-free nucleic acids from a predetermined source in a biological sample as described hereinbefore, by which the fraction of the cell-free nucleic acids from the predetermined source in the biological sample, especially the fraction of the cell-free fetal nucleic acids in a peripheral blood sample obtained from a pregnant woman, or the fraction of tumor-derived cell-free nucleic acids in a peripheral blood sample obtained from a subject suffering from tumor, suspected to suffer from tumor or subjected to tumor screening can be accurately and efficiently determined.

Additional aspects and advantages of embodiments of present disclosure will be given in part in the following descriptions, become apparent in part from the following descriptions, or be learned from the practice of the embodiments of the present disclosure.

## BRIEF DESCRIPTION OF THE DRAWINGS

These and other aspects and advantages of embodiments of the present disclosure will become apparent and more readily appreciated from the following descriptions made with reference to the drawings, in which:

Fig. 1 is a flow chart of a method for determining a fraction of cell-free nucleic acids from a predetermined source in a biological sample according to an embodiment of the present disclosure;

Fig. 2 is a flow chart for determining the number of sequencing reads falling into a predetermined window in the sequencing result according to an embodiment of the present disclosure;

Fig. 3 is a schematic diagram of a device for determining a fraction of cell-free nucleic acids from a predetermined source in a biological;

Fig. 4 is a schematic diagram of a counting apparatus 200 according to an embodiment of the present disclosure,

Fig. 5 is a weight distribution graph obtained by weight estimation using a ridge regression statistical model according to an embodiment of the present disclosure;

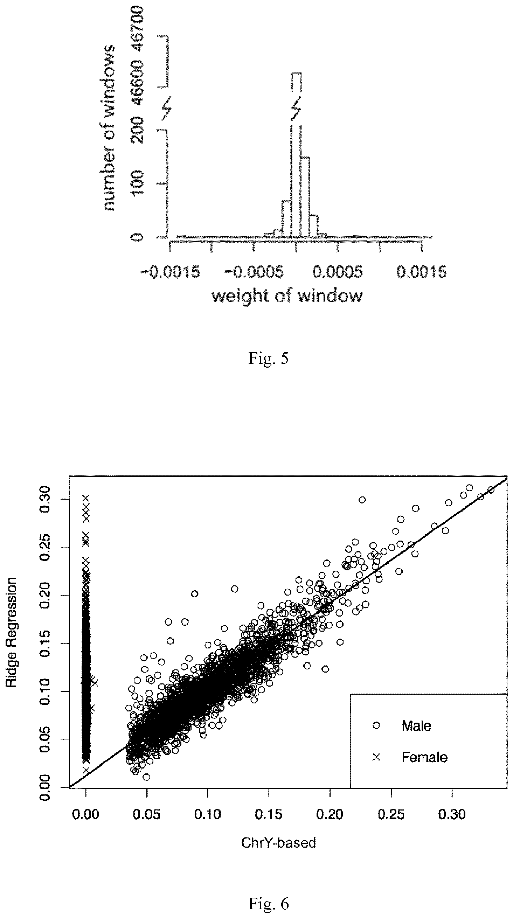

Fig. 6 is a graph showing correlation analysis between fetal fractions of samples in a testing set estimated by a ridge regression statistical model and fetal fractions estimated using Y chromosome according to an embodiment of the present disclosure, where Male represents a biological sample obtained from a pregnant woman with a male fetus, Female represents a biological sample obtained from a pregnant woman with a male fetus, ChrY-based represents a fraction of cell-free fetal nucleic acids estimated based on Y chromosome, and Ridge Regression represents a fraction of cell-free fetal nucleic acids estimated by the ridge regression statistical model according to an embodiment of the present disclosure;

Fig. 7 is a weight distribution graph obtained by weight estimation using a neural network model according to an embodiment of the present disclosure;

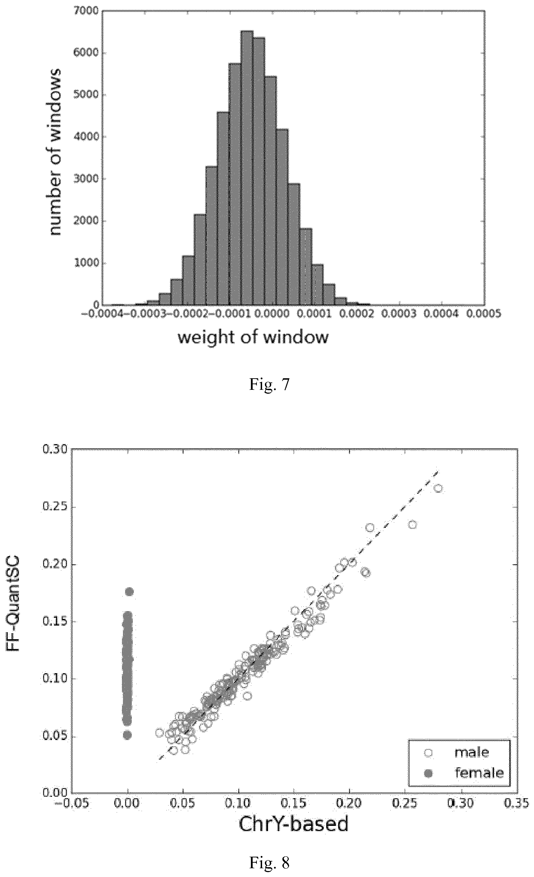

Fig. 8 is a graph showing correlation analysis between fetal fractions of samples in a testing set estimated by a neural network model and fetal fractions estimated using Y chromosome according to an embodiment of the present disclosure, where Male represents a biological sample obtained from a pregnant woman with a male fetus, Female represents a biological sample obtained from a pregnant woman with a female fetus, ChrY-based represents a fraction of cell-free fetal nucleic acids estimated based on Y chromosome, and FF-QuantSC represents a fraction of cell-free fetal nucleic acids estimated by the neural network model according to an embodiment of the present disclosure; and

Fig. 9 is a graph showing correlation analysis between female fetal fractions estimated by a ridge regression statistical model and that estimated by a neural network model according to an embodiment of the present disclosure, where Ridge Regression represents a fraction of cell-free fetal nucleic acids estimated by the ridge regression statistical model according to an embodiment of the present disclosure, and FF-QuantSC represents a fraction of cell-free fetal nucleic acids estimated by the neural network model according to an embodiment of the present disclosure.

## DETAILED DESCRIPTION

Embodiments of the present disclosure will be described in detail below, which are explanatory, illustrative, and used to generally explain the present disclosure, thus shall not be construed to limit the present disclosure.

### Method for determining a fraction of cell-free nucleic acids in a biological sample

According to embodiments of a first aspect of the present disclosure, a method for determining a fraction of cell-free nucleic acids from a predetermined source in a biological sample is provided. The inventors have surprisingly found that, the fraction of the cell-free nucleic acids in the biological sample, especially the fraction of cell-free fetal nucleic acids in a peripheral blood sample obtained from a pregnant woman, and the fraction of tumor derived nucleic acids in a peripheral blood sample obtained from a subject suffering from tumor can be accurately and efficiently determined by the method of the present disclosure.

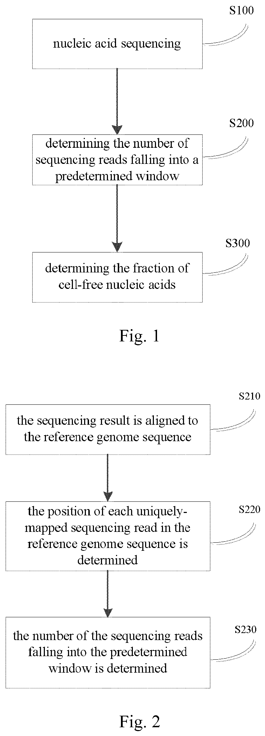

It should be noted that, expression "fraction of cell-free nucleic acids from a predetermined source in a biological sample" used herein refers to a fraction of the number of cell-free nucleic acids from a specific source to the total number of cell-free nucleic acids in the biological sample. For example, if the biological sample is a peripheral blood obtained from a pregnant woman, and the cell-free nucleic acids from the predetermined source are cell-free fetal nucleic acids, "a fraction of cell-free nucleic acids from a predetermined source in a biological sample" is a fraction of cell-free fetal nucleic acids, meaning a proportion of the number of cell-free fetal nucleic acid molecules to the total number of cell-free nucleic acid molecules in the peripheral blood obtained from the pregnant woman, which sometimes also may be known as "a fraction concentration of cell-free fetal DNA in the peripheral blood obtained from the pregnant woman" or "a proportion of cell-free fetal DNA" or "a percentage of cell-free fetal DNA". As another example, if the biological sample is a peripheral blood sample obtained from a subject suffering from tumor, suspected to suffer from tumor or subjected to tumor screening, and the cell-free nucleic acids from the predetermined source are tumor-derived cell-free nucleic acids, "a fraction of cell-free nucleic acids from the predetermined source in a biological sample" is a fraction of tumor-derived cell-free nucleic acids, meaning a proportion of the number of tumor-derived cell-free nucleic acids to the total number of cell-free nucleic acids in the peripheral blood sample obtained from the subject suffering from tumor, suspected to suffer from tumor or subjected to tumor screening. According to embodiments of the present disclosure and with reference to Fig. 1, the method includes the following steps.

### S100: nucleic acid sequencing

Cell-free nucleic acids in the biological sample are sequenced so as to obtain a sequencing result consisting of a plurality of sequencing reads.

In embodiments of the present disclosure, the biological sample is a peripheral blood sample. In embodiments of the present disclosure, the cell-free nucleic acid from the predetermined source is at least one selected from: cell-free fetal nucleic acids in a peripheral blood sample obtained from a pregnant woman; cell-free maternal nucleic acids in a peripheral blood sample obtained from a pregnant woman; and tumor-derived cell-free nucleic acids or non-tumor derived cell-free nucleic acids in a peripheral blood sample obtained from a subject suffering from tumor, suspected to suffer from tumor or subjected to tumor screening. Therefore, the fraction of cell-free fetal nucleic acids or cell-free maternal nucleic acids in the peripheral blood sample obtained from the pregnant woman, or the fraction of tumor-derived cell-free nucleic acids in the peripheral blood sample obtained from the subject suffering from tumor, suspected to suffer from tumor or subjected to tumor screening can be easily determined. In embodiments of the present disclosure, the cell-free nucleic acids in the biological sample are sequenced by paired-end sequencing, single-end sequencing or single molecule sequencing. In some specific embodiments of the present disclosure, the cell-free nucleic acids are DNAs. It should be noted that, term "sequencing data" used herein refers to "sequencing reads", which corresponds to nucleic acid molecules subjected to sequencing.

### S200: determining the number of sequencing reads falling into a predetermined window

The sequencing result is aligned to a reference sequence, so as to determine the number of sequencing reads falling into a predetermined window in the sequencing result.

In an embodiment of the present disclosure, the reference sequence is a reference genome sequence, preferably hg19.

In an embodiment of the present disclosure, the predetermined window is obtained by sequential division of a predetermined chromosome of the reference genome sequence.

In an embodiment of the present disclosure, the predetermined chromosome includes an autosome. Preferably, the autosome does not include any one of chromosomes 13, 18 and 21.

In an embodiment of the present disclosure, the predetermined window is of a length of 60Kbp.

It should be noted that, the division of the predetermined windows needs to keep reads uniformity within each window, i.e., ensure evenness of reads within each window. It should be illustrated that, "evenness of reads within each window" means that the number of reads in each window is substantially the same, i.e., a variance therebetween is close to 0.

According to embodiments of the present disclosure, referring to Fig. 2, **S200** further includes the followings.

At S210, the sequencing result is aligned to the reference genome sequence. Specifically, the sequencing result is aligned to the reference genome sequence, so as to construct a dataset consisting of a plurality of uniquely-mapped sequencing reads, where each uniquely-mapped sequencing read in the dataset can be mapped to a single position in the reference genome sequence.

At S220, the position of each uniquely-mapped sequencing read in the reference genome sequence is determined. Specifically, positions of individual uniquely-mapped sequencing reads in the reference genome sequence are determined.

At S230, the number of uniquely-mapped sequencing reads falling into the predetermined window is determined. In specific, the numbers of uniquely-mapped sequencing reads falling into individual predetermined windows are determined.

Therefore, it is easy to determine the number of uniquely-mapped sequencing reads falling into the predetermined window in the sequencing result, and the determined result is accurate, reliable and reproducible.

## S300: determining the fraction of cell-free nucleic acids

The fraction of the cell-free nucleic acids from the predetermined source in the biological sample is determined based on the number of the sequencing reads falling into the predetermined window.

In embodiments of the present disclosure, at S300, the fraction of the cell-free nucleic acids from the predetermined source in the biological sample is determined by a weight of each predetermined window. In some specific embodiments, at S300, the weight of each predetermined window is predetermined with training samples. Therefore, the result is accurate, reliable and repeatable. In some embodiments, the weight of each predetermined window is determined by at least one of a ridge regression statistical model and a neural network model. In some embodiments, the neural network model adopts a TensorFlow learning system. In some specific embodiments, the TensorFlow learning system includes the following parameters: the numbers of uniquely-mapped sequencing reads falling into individual windows of an autosome as an input layer; a fetal fraction as an output layer; ReLu as a neuron; an optimizer selected from at least one of Adam, SGD and Ftrl, preferably Ftrl. Preferably, the TensorFlow learning system further includes the following parameters: a learning rate set to be 0.002; 1 hidden layer; and 200 neurons in the hidden layer. Therefore, the result is accurate and reliable. It should be illustrated that, the term "weight" used herein is a relative concept for a certain index. A weight of a certain index refers to a relative importance of the index in the overall evaluation. For example, "a weight of a predetermined window" refers to a relative importance of the predetermined window in all predetermined windows. "A connection weight" refers to a relative importance of a certain two-layer connection in all two-layer connections.

In embodiments of the present disclosure, the training sample is a peripheral blood sample with a known fraction of cell-free fetal nucleic acids from a pregnant woman. Therefore, a fraction of cell-free fetal nucleic acids or cell-free maternal nucleic acids in a peripheral blood sample from a pregnant woman under detection may be effectively determined. In some specific embodiments, the training sample is a peripheral blood sample with a known fraction of cell-free fetal nucleic acids from a pregnant woman with a normal male fetus. Therefore, the determined result for the fraction of cell-free fetal nucleic acids or cell-free maternal nucleic acids in a peripheral blood sample from a pregnant woman under detection is more accurate and reliable.

In embodiments of the present disclosure, the weight of each predetermined window is determined by a ridge regression statistical model having the following computational formula:

$$\hat{y} = \hat{\beta}_0 + \sum_{j=1}^{p} \hat{\beta}_j x_j$$

where $\hat{y}$ is a predictive fetal fraction, $x_j$ is the number of uniquely-mapped sequencing reads falling into a window, $\hat{\beta}_j$ is a weight of a window, $\hat{\beta}_0$ is a deviation, and $\hat{\beta}_j$ and $\hat{\beta}_0$ are obtained by training the model.

In some embodiments of the present disclosure, the weight of each predetermined window is determined by a neural network model having the following computational formula:

$$z_j^l = f\left(\sum_k w_{jk}^l z_k^{l-1} + b_j^l\right)$$

,

where $l$ is a serial number of a layer in the neural network model, the first layer is an input layer, the last layer is an output layer (having only one neuron), and an intermediate layer is a hidden layer, $z_j^l$ is a value for a $j^{th}$ neuron in an $l^{th}$ layer, $z_k^{l-1}$ is a value for a $k^{th}$ neuron in an $(l-1)^{th}$ layer, $w_{jk}^l$ a connection weight from the $k^{th}$ neuron in the $(l-1)^{th}$ layer to the $j^{th}$ neuron in the $l^{th}$ layer, $b_j^l$ is an input deviation for the $j^{th}$ neuron in the $l^{th}$ layer, and w and b are obtained by training the model. A most common form of the function $f$ is a rectified linear unit, i.e., $f(x) = \max(0, x)$.

In some embodiments, when the neural network model is applied, values for individual neurons are calculated layer-by-layer based on the above computational formula of the neural network model, and a value for the neuron in the last layer is the predictive fetal fraction.

That is, according to embodiments of the present disclosure, determining the weight of each predetermined window by the neural network model includes: calculating values for individual neurons layer-by-layer according to the computational formula of the neural network model, where the value for the neuron in the last layer is the predictive fetal fraction.

In some embodiments, prior to S300, GC correction is performed on the number of uniquely-mapped sequencing reads falling into the predetermined window in advance, so as to obtain the number of GC corrected uniquely-mapped sequencing reads falling into the predetermined window. Therefore, the determined number of uniquely-mapped sequencing reads falling into the predetermined window is accurate and reliable.

Preferably, in some embodiments, the GC correction is performed by:

fitting the numbers of uniquely-mapped sequencing reads falling into individual predetermined windows of the predetermined chromosome with corresponding GC contents to determine $ER = f(gc)$;

performing correction on the number of uniquely-mapped sequencing reads for each predetermined window of all chromosomes: $ERA_i = ER_i * (\overline{ER}/f(GC_i))$, $i = 1,2,3, ,N$,

where for a sample, $ER_i$ represents the number of uniquely-mapped sequencing reads falling into an $i^{th}$ predetermined window, $GC_i$ represents a GC content of a reference sequence for the $i^{th}$ predetermined window, $\overline{ER}$ represents a mean value for the numbers of uniquely-mapped sequencing reads falling into individual windows of the predetermined chromosome; and $ERA_i$ represents the number of GC corrected uniquely-mapped sequencing reads in the $i^{th}$ predetermined window after correction.

In embodiments of the present disclosure, prior to S300, sex of the fetus is predetermined. Preferably, the sex of the fetus is determined by a ratio of the number of uniquely-mapped sequencing reads in Y chromosome to the total number of uniquely-mapped sequencing reads in all chromosomes. Therefore, the determined result for the fraction of cell-free fetal nucleic acids or cell-free maternal nucleic acids in a peripheral blood sample from a pregnant woman under detection is more accurate and reliable.

Further, according to some specific embodiments of the present disclosure, the estimation of a fetal fraction of a sequenced sample with the method of the present disclosure using the ridge regression statistical model or the neural network model in a TensorFlow library includes the following specific steps.

**1. Estimation of the fetal fraction of the sequenced sample using the ridge regression statistical model:**

1) sequentially dividing a reference sequence (hg19) into adjacent windows by a fixed length (such as 60Kbp in this embodiment), filtering out windows in an N region, and determining GC content for each window, so as to obtain a reference window file hg19.gc;

2) alignment: aligning sequencing reads (each in 28bp) sequenced by single-end (SE) sequencing on CG platform to the reference sequence (hg19) using such as BWA V0.7.7-r441;

3) filtration and preliminary statistics: selecting uniquely-mapped sequencing reads from alignment results, filtering out repeated reads and base-mismatched reads to obtain effective sequencing reads, and determining the number of effective sequencing reads falling into each window of the reference window file hgl9.gc and corresponding GC contents;

4) GC correction, including the following specific steps:

fitting the numbers of effective sequencing reads falling into individual windows of an autosome with corresponding GC contents (such as using cubic spline fitting in this embodiment) to determine ER = f(gc);

performing correction on the number of effective sequencing reads for each window of all chromosomes: $ERA_t = ER_i * (\overline{ER}/f(GC_i))$, $i = 1,2,3,... , N,$

where for a sample, $ER_i$ represents the number of effective sequencing reads falling into an $i^{th}$ window, $GC_i$ represents a GC content of the reference sequence for the $i^{th}$ window (records in the reference window file hg19.gc), $\overline{ER}$ represents a mean value for the numbers of effective sequencing reads falling into individual windows of an autosome (such as chromosomes 1-22); and $ERA_i$ represents the number of GC corrected effective sequencing reads in the $i^{th}$ window after the GC correction;

5) determining sex of a fetus: comparing a ratio (ER%) of the number of GC corrected effective sequencing reads in Y chromosome to the total number of GC corrected effective sequencing reads in all chromosomes with a specified threshold $a$ within a range of [0.001, 0.003], determining the fetus to be a male fetus if the ER% is greater than or equal to $a$, otherwise determining the fetus to be a female fetus if the ER% is less than $a$;

6) estimating the fetal fraction using the ridge regression statistical model, including the following steps:

a) selecting samples each independently obtained from a pregnant woman with a male fetus as a training set; and selecting a batch of samples as a testing set (in which the number of samples each independently obtained from a pregnant woman with a male fetus may be the same as that from a pregnant woman with a female fetus);

b) estimating the weight of each window in the autosome (excluding chromosomes 13, 18 and 21) using the ridge regression model (the weight is equivalent to a regression coefficient β in the ridge regression model) for the training set, to obtain an estimated weight distribution; and

c) estimating the fetal fraction of the sample in the testing set with the known weights.

**2. Estimation of the fetal fraction of the sequenced sample using the neural network model in the TensorFlow library:**

The first five steps 1)-5) are the same as that described above in the ridge regression statistical model;

6) estimating the fetal fraction using the neural network model in the TensorFlow library, including the following specific steps:

a) selecting samples each independently obtained from a pregnant woman with a male fetus as a training set; and selecting a batch of samples as a testing set (in which the number of samples each independently obtained from a pregnant woman with a male fetus may be the same as that from a pregnant woman with a female fetus), standardizing all the data (all of the numbers of GC corrected effective sequencing reads falling into individual windows), i.e., linearly transforming each variable (the number of GC corrected effective sequencing reads falling into each window) so that a mean value for the variables in all the samples is 0, and a standard deviation is 1;

b) constructing a neural network in which the standardized numbers of effective sequencing reads falling into individual windows of an relatively stable autosome are taken as an input layer, a single neuron is taken as an output layer (corresponding to the fetal fraction), there is no hidden layer, ReLU is selected as the neuron type and Adam as an optimizer;

c) applying the neural network (learning)in the training set to predict the fetal fraction, and adjusting a learning rate according to the change of learning effect in each round, so as to maximize the learning rate while ensuring the learning effect for the training set without repeat fluctuation ;

d) training as many rounds as the computational ability allows, until the learning effect is saturated;

e) switching to other optimizers (such as SGD, Ftrl, *etc.*), repeating the steps b)-d), and selecting an optimal optimizer based on the learning effects;

f) attempting to add a second-order regularization term to the neural network model and adjusting its size to observe the learning effects before and after the addition of and the size adjustment of the second-order regularization term;

g) adding a hidden layer, adjusting the number of neurons in the hidden layer, repeating the steps b)-f), and selecting an optimal hidden layer architecture based on the learning effects;

h) training the optimized neural network model on the training set to obtain optimal parameters and a distribution of weights (average weights of neurons in the input layer to the hidden layer) of individual windows; and

i) estimating the fetal fraction of the sample in the testing set with the trained neural network model.

To facilitate understanding, basic principles of the models described in the present disclosure are briefly introduced as follows.

(1) Ridge regression statistical model

Ridge regression is a modified least square method, which reduces over-fitting of the model by adding the second-order regularization term.

In mathematical form, the least square method is to solve $\beta_0$, $\beta_1$, $\beta_2$, ..., which minimize a sum of squares of residuals:

$$\text{RSS} = \sum_{i=1}^{n} \left( y_i - \beta_0 - \sum_{j=1}^{p} \beta_j x_{ij} \right)^2$$

where RSS is the sum of squares of residuals, $y_i$ is a dependent variable, and $x_{ij}$ is an independent variable.

If the above function is modified by adding the second-order regularization term, the ridge regression is obtained, which is to solve $\beta_0$, $\beta_1$, $\beta_2$, ... *to* minimize the following function:

$$\sum_{i=1}^{n} \left( y_i - \beta_0 - \sum_{j=1}^{p} \beta_j x_{ij} \right)^2 + \lambda \sum_{j=1}^{p} \beta_j^2 = \text{RSS} + \lambda \sum_{j=1}^{p} \beta_j^2 \text{ ,}$$

where $\lambda$ needs to be specified, and a general practice to specify the $\lambda$ is to take several values and determine which $\lambda$ minimizes the objective function of the validation set through cross-validation.

(2) Artificial Neural Network

Artificial neural network (i.e., the neural network model) is a non-linear machine learning method. Its basic elements are neurons, and each neuron performs weighted averaging with a deviation on several inputs $x_j$:

$$z = \sum_j (w_j x_j + w_0) \text{ ,}$$

where $w_j$ is a weight, and $w_0$ is the deviation,

$f(z)$ is an output depending on the result of weighted averaging. At present, the most commonly used function form is the rectified linear unit, i.e., $f(z) = max(0,z)$.

Several neurons constitute a multi-layer network, in which the first layer (i.e., the input layer) takes the standardized numbers of effective sequencing reads falling into individual predetermined windows as an independent variable for input, while an output from a former layer is taken as an input for a latter layer, running like this until the last layer (i.e., output layer) which has only one neuron and one output (i.e., a model predicted value). A layer other than the input and output layers is called the hidden layer.

Basic parameters of the neural network model include the weight of each layer and the deviation, which are generally trained by backpropagation. In addition, there are other parameters such as the learning rate, the neuron type, the optimization algorithm (optimizer), the number of hidden layers, the number of neurons in the hidden layer, regularization coefficient and so on, which are generally preset according to experiences and adjusted repeatedly based on the training effects.

**Device for determining a fraction of cell-free nucleic acids in a biological sample**

According to embodiments of a second aspect of the present disclosure, there is further provided Also disclosed, but not part of the invention, is a device for determining a fraction of cell-free nucleic acids from a predetermined source in a biological sample. The inventors have surprisingly found that, the device of the present disclosure is suitable to carry out the method for determining a fraction of cell-free nucleic acids from a predetermined source in a biological sample as described hereinbefore, by which the fraction of the cell-free nucleic acids from the predetermined source in the biological sample, especially the fraction of the cell-free fetal nucleic acids or cell-free maternal nucleic acids in a peripheral blood sample obtained from a pregnant woman, or the fraction of tumor-derived cell-free nucleic acids in a peripheral blood sample obtained from a subject suffering from tumor, suspected to suffer from tumor or subjected to tumor screening can be accurately and efficiently determined.

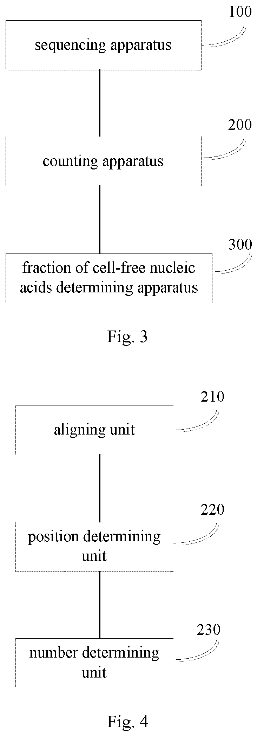

Referring to Fig. 3, the device includes: a sequencing apparatus 100, a counting apparatus 200 and a fraction of cell-free nucleic acids determining apparatus 300.

Specifically, the sequencing apparatus 100 is configured to perform sequencing on cell-free nucleic acids contained in the biological sample, so as to obtain a sequencing result consisting of a plurality of sequencing reads. The counting apparatus 200 is connected to the sequencing apparatus 100 and configured to align the sequencing result to a reference sequence, so as to determine the number of sequencing reads falling into a predetermined window in the sequencing result. The fraction of cell-free nucleic acids determining apparatus 300 is connected to the counting apparatus 200 and configured to determine the fraction of the cell-free nucleic acids from the predetermined source in the biological sample based on the number of the sequencing reads falling into the predetermined window.

According to embodiments of the present disclosure, the biological sample is not limited to a specific type. In a specific embodiment, the biological sample is a peripheral blood sample. In an embodiment, the cell-free nucleic acid from the predetermined source is at least one selected from the followings: cell-free fetal nucleic acids in a peripheral blood sample obtained from a pregnant woman; cell-free maternal nucleic acids in a peripheral blood sample obtained from a pregnant woman; and tumor-derived cell-free nucleic acids or non-tumor derived cell-free nucleic acids in a peripheral blood sample obtained from a subject suffering from tumor, suspected to suffer from tumor or subjected to tumor screening. Therefore, the fraction of cell-free fetal nucleic acids or cell-free maternal nucleic acids in the peripheral blood sample obtained from the pregnant woman, or the fraction of tumor-derived cell-free nucleic acids in the peripheral blood sample obtained from the subject suffering from tumor, suspected to suffer from tumor or subjected to tumor screening can be easily determined.

In embodiments of the present disclosure, the cell-free nucleic acids are DNAs.

In embodiments of the present disclosure, the cell-free nucleic acids in the biological sample are sequenced by paired-end sequencing, single-end sequencing or single molecule sequencing.

In embodiments of the present disclosure, the reference sequence is a reference genome sequence, preferably hg19.

In embodiments of the present disclosure, the predetermined window is obtained by sequential division of a predetermined chromosome of the reference genome sequence. According to some embodiments of the present disclosure, the predetermined chromosome includes an autosome. Preferably, the autosome does not include any one of chromosomes 13, 18 and 21. In a preferred embodiment of the present disclosure, the predetermined window is of a length of 60Kbp.

In embodiments of the present disclosure, referring to Fig. 4, the counting apparatus 200 further includes: an aligning unit 210, a position determining unit 220 and a number determining unit 230. Specifically, the aligning unit 210 is configured to align the sequencing result to the reference genome sequence, so as to construct a dataset consisting of a plurality of uniquely-mapped sequencing reads, where each uniquely-mapped sequencing read in the dataset can be mapped to a single position in the reference genome sequence. The position determining unit 220 is connected to the aligning unit 210 and configured to determine the position of each uniquely-mapped sequencing read in the reference genome sequence. The number determining unit 230 is connected to the position determining unit 220 and configured to determine the number of uniquely-mapped sequencing reads falling into the predetermined window. Therefore, it is easy to determine the number of uniquely-mapped sequencing reads falling into the predetermined window, and the determined result is accurate, reliable and reproducible.

According to embodiments of the present disclosure, the fraction of cell-free nucleic acids determining apparatus 300 is suitable to determine the fraction of the cell-free nucleic acids from the predetermined source in the biological sample by a weight of each predetermined window. In some specific embodiments, the weight of each predetermined window is predetermined with training samples. Therefore, the result is accurate, reliable and repeatable. In an embodiment, the weight of each predetermined window is determined by at least one of a ridge regression statistical model and a neural network model. In some embodiments, the neural network model adopts a TensorFlow learning system. In some specific embodiments, the TensorFlow learning system includes the following parameters: the number of uniquely-mapped sequencing reads falling into individual windows of an autosome as an input layer; a fetal fraction as an output layer; ReLu as a neuron; an optimizer selected from at least one of Adam, SGD and Ftrl, preferably Ftrl. Preferably, the TensorFlow learning system further includes the following parameters: a learning rate set to be 0.002; 1 hidden layer; and 200 neurons in the hidden layer. Therefore, the result is accurate and reliable.

In embodiments of the present disclosure, the training sample is a peripheral blood sample with a known fraction of cell-free fetal nucleic acids from a pregnant woman. Therefore, a fraction of cell-free fetal nucleic acids or cell-free maternal nucleic acids in a peripheral blood sample from a pregnant woman under detection may be effectively determined. In some specific embodiments, the training sample is a peripheral blood sample with a known fraction of cell-free fetal nucleic acids from a pregnant woman with a normal male fetus. Therefore, the determined result for the fraction of cell-free fetal nucleic acids or cell-free maternal nucleic acids in a peripheral blood sample from a pregnant woman under detection is more accurate and reliable.

In embodiments of the present disclosure, the weight of each predetermined window is determined by a ridge regression statistical model having the following computational formula:

$$\hat{y} = \hat{\beta}_0 + \sum_{j=1}^{p} \hat{\beta}_j x_j ,$$

where $\hat{y}$ is a predictive fetal fraction, $x_j$ is the number of uniquely-mapped sequencing reads falling into a window, $\hat{\beta}_j$ is a weight of a window, $\hat{\beta}_0$ is a deviation, and $\hat{\beta}_j$ and $\hat{\beta}_0$ are obtained by training the model.

In some embodiments of the present disclosure, the weight of each predetermined window is determined by a neural network model having the following computational formula:

$$z_j^l = f\left(\sum_k w_{jk}^l z_k^{l-1} + b_j^l\right) ,$$

where $l$ is a serial number of a layer in the neural network model, the first layer is an input layer, the last layer is an output layer (having only one neuron), and an intermediate layer is a hidden layer, $z_j^l$ is a value for a $j^{th}$ neuron in an $l^{th}$ layer, $z_k^{l-1}$ is a value for a $k^{th}$ neuron in an $(l-1)^{th}$ layer, $w_{jk}^l$ a connection weight from the $k^{th}$ neuron in the $(l-1)^{th}$ layer to the $j^{th}$ neuron in the $l^{th}$ layer, $b_j^l$ is an input deviation for the $j^{th}$ neuron in the $l^{th}$ layer, and w and b are obtained by training the model. A most common form of the function $f$ is a rectified linear unit, i.e., $f(x) = \max(0,x)$.

In some embodiments, determining the weight by the neural network model includes: calculating values for individual neurons layer-by-layer according to the computational formula of the neural network model, where the value for the neuron in the last layer is the predictive fetal fraction.

The disclosed device further includes a GC correction apparatus (not shown in figures) connected to each of the counting apparatus 200 and the fraction of cell-free nucleic acids determining apparatus 300 and configured to perform GC correction on the number of uniquely-mapped sequencing reads falling into the predetermined window in advance, so as to obtain the number of GC corrected uniquely-mapped sequencing reads falling into the predetermined window, prior to determining the fraction of the cell-free nucleic acid from the predetermined source in the biological sample. Therefore, the determined number of uniquely-mapped sequencing reads falling into the predetermined window is accurate and reliable.

Preferably, in some embodiment of the present disclosure, the GC correction apparatus is suitable to perform the GC correction in accordance with the following operations:

fitting the numbers of uniquely-mapped sequencing reads falling into individual predetermined windows of the predetermined chromosome with corresponding GC contents to determine $ER = f(gc)$;

performing correction on the number of uniquely-mapped sequencing reads for each predetermined window of all chromosomes: $ERA_i = ER_i * (\overline{ER}/f(GC_i))$, $i = 1,2,3, \ldots ,N,$

where for a sample, $ER_i$ represents the number of uniquely-mapped sequencing reads falling into an $i^{th}$ predetermined

window, $GC_i$ represents a GC content of a reference sequence for the $i^{th}$ predetermined window, $\overline{ER}$ represents a mean value for the numbers of uniquely-mapped sequencing reads falling into individual windows of the predetermined chromosome; and $ERA_i$ represents the number of GC corrected uniquely-mapped sequencing reads in the $i^{th}$ predetermined window after correction.

The disclosed device may further include a sex determining apparatus (not shown in figures) connected to the fraction of cell-free nucleic acids determining apparatus 300 and configured to predetermine sex of the fetus. Preferably, the sex of the fetus is determined by a ratio of the number of uniquely-mapped sequencing reads in Y chromosome to the total number of uniquely-mapped sequencing reads in all chromosomes. Therefore, the determined result for the fraction of cell-free fetal nucleic acids or cell-free maternal nucleic acids in a peripheral blood sample from a pregnant woman under detection is more accurate and reliable.

It should be noted that, the expression "a normal male fetus/female fetus/fetus" means that the fetus having normal chromosomes, for example, "a normal male fetus" refers to a male fetus with normal chromosomes. Moreover, the expression "a normal male fetus/female fetus/fetus" may refer to a single fetus or twins, for example, "a normal male fetus" may be a normal single fetus or normal twins; and "a normal fetus" neither limits the sex of the fetus nor limits the fetus being single fetus or twins.

Embodiments of the present disclosure will be described in detail below with reference to examples. It should be appreciated to those skilled in the art that the following examples are merely used to illustrate the present disclosure, and thus shall not be construed to limit the scope of the present disclosure. An example without specified conditions will be carried out under normal conditions or as recommended by the manufacturer. Reagents or instruments not specifying the manufacturers are conventional products available from the market.

**Example 1**

The fetal fraction was estimated for 1400 sequenced samples using the ridge regression statistical model according to the following steps:

 1) sequentially dividing a reference sequence (hg19) into adjacent windows by a fixed length (such as 60Kbp in this example), filtering out windows in an N region, and determining GC content for each window, so as to obtain a reference window file hg19.gc;
 2) alignment: aligning sequencing reads (each in 28 bp) sequenced by SE sequencing on CG platform to the reference sequence (hg19) using such as BWA V0.7.7-r441;
 3) filtration and preliminary statistics: selecting uniquely-mapped sequencing reads from alignment results, filtering out repeated reads and base-mismatched reads to obtain effective sequencing reads, and determining the number of effective sequencing reads falling into each window of the reference window file hgl9.gc and corresponding GC contents;
 4) GC correction, including the following specific steps:

  fitting the numbers of effective sequencing reads falling into individual windows of an autosome and corresponding GC contents (such as using cubic spline fitting in this example) to determine $\overline{ER} = f(gc)$;

  performing correction on the number of effective sequencing reads for each window of all chromosomes: $ERA_i = ER_i * (\overline{ER}/f(GC_i))$, $i = 1,2,3, ... , N$,

  where for a sample, $ER_i$ represents the number of effective sequencing reads falling into an $i^{th}$ window, $GC_i$ represents a GC content of the reference sequence in the $i^{th}$ window (records in the reference window file hg19.gc), $\overline{ER}$ represents a mean value for the numbers of effective sequencing reads falling into individual windows of an autosome (such as chromosomes 1-22); and $ERA_i$ represents the number of GC corrected effective sequencing reads in the $i^{th}$ window after the GC correction;

 5) determining sex of a fetus: comparing a ratio (ER%) of the number of GC corrected effective sequencing reads in Y chromosome to the total number of GC corrected effective sequencing reads in all chromosomes with a specified threshold of [0.001, 0.003], determining the fetus to be a male fetus if the ER% is greater than or equal to 0.003, and determining the fetus to be a female fetus if the ER% is less than 0.001;
 6) estimating the fetal fraction using the ridge regression statistical model, including the following steps:

a) selecting 1000 samples each independently obtained from a pregnant woman with a male fetus as a training set; and selecting 400 samples as a testing set of which 200 samples are each independently obtained from a pregnant woman with a male fetus and 200 samples are each independently obtained from a pregnant woman with a female fetus;

b) estimating a weight of each window in the autosome (excluding chromosomes 13, 18 and 21) using the ridge regression mode for the training set, i.e., determining a deviation $\beta_0$ and a weight $\beta_j$ that minimize the following formula:

$$\sum_{i=1}^{n}\left(y_i - \beta_0 - \sum_{j=1}^{p}\beta_j x_{ij}\right)^2 + \lambda\sum_{j=1}^{p}\beta_j^2 = \text{RSS} + \lambda\sum_{j=1}^{p}\beta_j^2 \quad,$$

$$\text{RSS} = \sum_{i=1}^{n}\left(y_i - \beta_0 - \sum_{j=1}^{p}\beta_j x_{ij}\right)^2$$

where                                                          , $y_i$ is a fetal fraction estimated with the Y chromosome, $x_{ij}$ is the number of GC corrected effective sequencing reads falling into a window, and $\lambda$ is a coefficient of a second-order regularization term, which is determined to minimize the objective function of validation set through cross-validation, the distribution of estimated weights being shown in Fig. 5; and

c) estimating the fetal fraction of the sample in the testing set with the known weights in accordance with the following formula:

$$\hat{y} = \hat{\beta}_0 + \sum_{j=1}^{p}\hat{\beta}_j x_j$$

where $\hat{y}$ is a predictive fetal fraction, $x_j$ is the number of GC corrected effective sequencing reads falling into a window, $\hat{\beta}_j$ is a weight of a window, $\hat{\beta}_0$ is a deviation, and $\hat{\beta}_j$ and $\hat{\beta}_0$ are obtained by training the model.

A correlation between the fetal fractions estimated by the ridge regression statistical model and that estimated by Y chromosome is shown in Fig. 6, from which it can be seen that there is a strong correlation therebetween (r = 0.92; p value < 1e-10), indicating that the fraction of cell-free fetal nucleic acids estimated by the method according to embodiments of the present disclosure is accurate and reliable.

**Example 2**

The fetal fraction was estimated for 1400 sequenced samples using the neural network model in the TensorFlow library according to the following steps:

steps 1)-5) being the same as that described above in Example 1;

6) estimating the fetal fraction using the neural network model in the TensorFlow library, including the following specific steps:

a) selecting 1000 samples each independently obtained from a pregnant woman with a male fetus as a training set; and selecting 400 samples as a testing set of which 200 samples are each independently obtained from a pregnant woman with a male fetus and 200 samples are each independently obtained from a pregnant woman with a female fetus, standardizing all the data (all of the numbers of GC corrected effective sequencing reads falling into individual windows), i.e., linearly transforming each variable (the number of GC corrected effective sequencing reads falling into each window) so that a mean value for the variables in all the samples is 0, and a standard deviation is 1;

b) constructing a neural network model in which the standardized number of effective sequencing reads falling into each window of an relatively stable autosome is taken as an input layer, a single neuron is taken as an output layer (corresponding to the fetal fraction), there is no hidden layer, ReLU is selected as the neuron type and Adam as an optimizer;

c) applying the neural network model (learning) in the training set to predict the fetal fraction, and adjusting a learning rate according to the change of learning effect in each round, so as to maximize the learning rate while ensuring the learning effect for the training set without repeat fluctuation, in which the learning method is to calculate a value $z_k^l$ for each neuron layer-by-layer in accordance with the following formula based on the standardized number $z_k^1$ of effective sequencing reads falling into each window:

$$z_j^l = f(\sum_k w_{jk}^l z_k^{l-1} + b_j^l)$$

,

where $l$ is a serial number of a layer in the neural network model, the first layer is an input layer, the last layer is an output layer (having only one neuron), and an intermediate layer is a hidden layer, $z_j^l$ is a value for a $j^{th}$ neuron in an $l^{th}$ layer, $z_k^{l-1}$ is a value for a $k^{th}$ neuron in an $(l-1)^{th}$ layer, $w_{jk}^l$ a connection weight from the $k^{th}$ neuron in the $(l-1)^{th}$ layer to the $j^{th}$ neuron in the $l^{th}$ layer, $b_j^l$ is an input deviation for the $j^{th}$ neuron in the $l^{th}$ layer, and a most common form of the function $f$ is a rectified linear unit, i.e., $f(x) = \max(0,x)$;

for each sample {$s$}, comparing a value $z_s^L$ for a neuron in the output layer with a fetal fraction $y_s$ estimated by the Y chromosome, and adjusting the connection weight $w_{jk}^l$ and the deviation $b_j^l$ for each layer to minimize $\sum_s (z_s^L - y_s)^2$ ;

d) training as many rounds as the computational ability allows, until the learning effect is saturated;

e) switching to other optimizers (such as SGD, Ftrl, *etc.*), repeating the steps b)-d), and selecting an optimal optimizer based on the learning effects;

f) attempting to add a second-order regularization term λ to the neural network model and adjusting its size to observe the learning effects before and after the addition of and size adjustment of the second-order regularization term, where the significance of the second-order regularization term is that it is no longer to seek minimum of $\sum_s (z_s^L - y_s)^2$ ; but to seek minimum of $\sum_s (z_s^L - y_s)^2 + \lambda \sum w^2$ in the learning process;

g) adding a hidden layer, adjusting the number of neurons in the hidden layer, repeating the steps b)-f), and selecting an optimal hidden layer architecture based on the learning effects;

h) training the optimized neural network model on the training set to obtain optimal parameters as shown in Table 1 and a distribution of weights (average weights of neurons in input layer to the hidden layer) of individual windows (referring to Fig. 7); and

i) estimating the fetal fraction of the sample in the testing set with the trained neural network model by calculating a value zk for each neuron layer-by-layer in accordance with the following formula based on the standardized number $z_k^1$ of effective sequencing reads falling into each window, and a value for a neuron in the last layer being the predictive fetal fraction:

$$z_j^l = f(\sum_k w_{jk}^l z_k^{l-1} + b_j^l)$$

.

Table 1 Optimal parameters obtained by the neural network model in TensorFlow library

| Learning rate | Optimizer | Second-order regularization strength | Number of hidden laver | Number of neurons in the hidden layer |
|---|---|---|---|---|
| 0.002 | Ftrl | 0 | 1 | 200 |

A correlation between the fetal fractions estimated by the neural network model and that estimated by Y chromosome is shown in Fig. 8 from which it can be seen that there is a strong correlation therebetween (r = 0.982; p value < 1e-10), indicating that the fraction of cell-free fetal nucleic acids estimated by the method according to embodiments of the present disclosure is accurate and reliable.

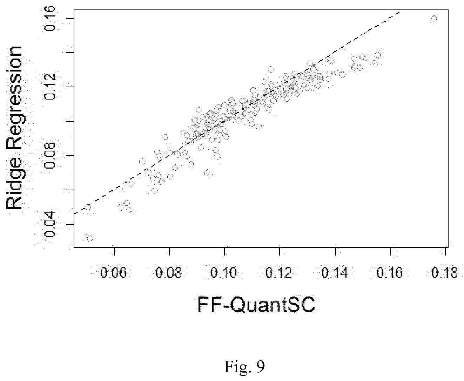

Finally, the inventors analyzed a correlation between the ridge regression statistical model and the neural network model in terms of estimation for female fetal fraction, the result is shown in Fig. 9, from which it can be clearly seen that the fetal fractions obtained by the two models are highly correlated (r = 0.935; p value < 1e-10).

Reference throughout this specification to "an embodiment", "some embodiments", "one embodiment", "another example", "an example", "a specific example", or "some examples", means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example", or "in some examples", in various places throughout this specification are not necessarily referring to the same embodiment or example of the present disclosure. Furthermore, the particular features, structures, materials, or characteristics may be combined in any suitable manner in one or more embodiments or examples.

## Claims

1. A method for determining a fraction of cell-free nucleic acids from a predetermined source in a biological sample, comprising:

   (1) performing sequencing on cell-free nucleic acids contained in the biological sample, so as to obtain a sequencing result consisting of a plurality of sequencing reads;
   (2) aligning the sequencing result to a reference sequence, so as to determine the number of sequencing reads falling into a predetermined window in the sequencing result,

      wherein the reference sequence is a reference genome sequence,
      wherein the predetermined window is obtained by sequential division of a predetermined chromosome of the reference genome sequence; and

   (3) determining the fraction of the cell-free nucleic acids from the predetermined source in the biological sample based on the number of the sequencing reads falling into the predetermined window, wherein the fraction of the cell-free nucleic acids from the predetermined source in the biological sample is determined by a weight of each predetermined window,

      wherein the weight of each predetermined window is predetermined with training samples and is determined by at least one of a ridge regression statistical model and a neural network model,
      wherein the training sample is a peripheral blood sample with a known fraction of cell-free fetal nucleic acids from a pregnant woman,
      wherein the training sample is a peripheral blood sample with a known fraction of cell-free fetal nucleic acids from a pregnant woman with a normal male fetus, where the normal male fetus refers to a male fetus with normal chromosomes,
      wherein the biological sample is a peripheral blood sample,
      wherein the cell-free nucleic acid from the predetermined source is at least one selected from the followings:

         cell-free fetal nucleic acids in a peripheral blood sample that has been obtained from a pregnant woman; and
         cell-free maternal nucleic acids in a peripheral blood sample that has been obtained from a pregnant woman.

**2.** The method according to claim 1, wherein the predetermined chromosome comprises an autosome, preferably, wherein the autosome does not comprise any one of chromosomes 13, 18 and 21.

**3.** The method according to any one of claims 1 or 2, wherein step (2) further comprises:

(2-1) aligning the sequencing result to the reference genome sequence, so as to construct a dataset consisting of a plurality of uniquely-mapped sequencing reads, where each uniquely-mapped sequencing read in the dataset maps to a single position in the reference genome sequence;
(2-2) determining the position of each uniquely-mapped sequencing read in the reference genome sequence; and
(2-3) determining the number of uniquely-mapped sequencing reads falling into the predetermined window.

**4.** The method according to any one of claims 1 to 3, wherein at step (3),

the neural network model adopts a TesnsorFlow learning system,
preferably, wherein the TensorFlow learning system comprises the following parameters:

the numbers of uniquely-mapped sequencing reads falling into individual windows of an autosome as an input layer;
a fetal fraction as an output layer;
ReLu as a neuron;
an optimizer selected from at least one of Adam, SGD and Ftrl,
preferably, wherein the optimizer is Ftrl,
preferably, wherein the TensorFlow learning system further comprises the following parameters:

a learning rate set to be 0.002;
1 hidden layer; and
200 neurons in the hidden layer.

**5.** The method according to claim 4, wherein the weight of each predetermined window is determined by a ridge regression statistical model having the following computational formula:

$$\hat{y} = \hat{\beta}_0 + \sum_{j=1}^{p} \hat{\beta}_j x_j$$

where $\hat{y}$ is a predictive fetal fraction, $x_j$ is the number of uniquely-mapped sequencing reads falling into a window, $\hat{\beta}_j$ is a weight of a window, $\hat{\beta}_0$ is a deviation, $\hat{\beta}_j$ and $\hat{\beta}_0$ are obtained by training the model.

**6.** The method according to claim 4, wherein the weight of each predetermined window is determined by a neural network model having the following computational formula:

$$z_j^l = f\left(\sum_k w_{jk}^l z_k^{l-1} + b_j^l\right)$$

where $l$ is a serial number of a layer in the neural network model, zj is a value for a $j^{th}$ neuron in an $l^{th}$ layer, $z_k^{l-1}$ is a value for a $k^{th}$ neuron in an $(l-1)^{th}$ layer, $w_{jk}^l$ a connection weight from the $k^{th}$ neuron in the $(l-1)^{th}$ layer to the $j^{th}$ neuron in the $l^{th}$ layer, $b_j^l$ is an input deviation for the $j^{th}$ neuron in the $l^{th}$ layer, and w and b are obtained by training the model,
preferably, wherein determining the weight of each predetermined window determined by the neural network model comprises:
calculating a value for each neurons layer-by-layer according to the computational formula of the neural network model, where a value for a neuron in the last layer is the predictive fetal fraction.

7. The method according to any one of claims 1 to 6, wherein prior to step (3), GC correction is performed on the number of uniquely-mapped sequencing reads falling into the predetermined window in advance, so as to obtain the number of GC corrected uniquely-mapped sequencing reads falling into the predetermined window, preferably, wherein the GC correction is performed by:

fitting the numbers of uniquely-mapped sequencing reads falling into individual predetermined windows of the predetermined chromosome with corresponding GC contents to determine $ER = f(gc)$;
performing correction on the number of uniquely-mapped sequencing reads for each predetermined window of the predetermined chromosome:

$$ERA_i = ER_i * (\overline{ER}/f(GC_i)), \quad i = 1,2,3,\dots,N,$$

where for a sample, $ER_i$ represents the number of uniquely-mapped sequencing reads falling into an $i^{th}$ predetermined window, $GC_i$ represents a GC content of a reference sequence for the $i^{th}$ predetermined window, $\overline{ER}$ represents a mean value for the numbers of uniquely-mapped sequencing reads falling into individual predetermined windows of the predetermined chromosome; and $ER_{A_i}$ represents the number of GC corrected uniquely-mapped sequencing reads in the $i^{th}$ predetermined window after correction.

8. The method according to any one of claims 1 to 7, wherein prior to step (3), sex of the fetus is predetermined, preferably, wherein the sex of the fetus is determined by a ratio of the number of uniquely-mapped sequencing reads in Y chromosome to the total number of uniquely-mapped sequencing reads in all chromosomes.

**Patentansprüche**

1. Ein Verfahren zur Bestimmung eines Anteils an zellfreien Nukleinsäuren aus einer vorbestimmten Quelle in einer biologischen Probe, umfassend:

(1) Durchführen einer Sequenzierung von zellfreien Nukleinsäuren, die in der biologischen Probe enthalten sind, um ein Sequenzierungsergebnis zu erhalten, das aus einer Vielzahl von Sequenzierungs-Reads besteht;
(2) Alignment des Sequenzierungsergebnisses an einer Referenzsequenz, um die Anzahl der Sequenzierungs-Reads zu bestimmen, die in ein vorbestimmtes Fenster in dem Sequenzierungsergebnis fallen,

wobei die Referenzsequenz eine Referenzgenomsequenz ist,
wobei das vorbestimmte Fenster durch sequenzielle Teilung eines vorbestimmten Chromosoms der Referenzgenomsequenz erhalten wird; und

(3) Bestimmen des Anteils der zellfreien Nukleinsäuren aus der vorbestimmten Quelle in der biologischen Probe auf der Grundlage der Anzahl der Sequenzierungs-Reads, die in das vorbestimmte Fenster fallen, wobei der Anteil der zellfreien Nukleinsäuren aus der vorbestimmten Quelle in der biologischen Probe durch eine Gewichtung jedes vorbestimmten Fensters bestimmt wird,

wobei die Gewichtung jedes vorbestimmten Fensters mit Trainingsproben vorbestimmt ist und durch mindestens eines von einem statistischen Ridge-Regressionsmodell und einem neuronalen Netzwerkmodell bestimmt wird,
wobei die Trainingsprobe eine Peripherblutprobe mit einem bekannten Anteil an zellfreien fetalen Nukleinsäuren von einer schwangeren Frau ist,
wobei die Trainingsprobe eine Peripherblutprobe mit einem bekannten Anteil an zellfreien fetalen Nukleinsäuren von einer schwangeren Frau mit einem normalen männlichen Fötus ist, wobei sich der normale männliche Fötus auf einen männlichen Fötus mit normalen Chromosomen bezieht,
wobei die biologische Probe eine Peripherblutprobe ist,
wobei die zellfreie Nukleinsäure aus der vorbestimmten Quelle mindestens eine aus den folgenden ausgewählt ist:

zellfreie fetale Nukleinsäuren in einer Peripherblutprobe, die von einer schwangeren Frau erhalten wurde; und

zellfreie mütterliche Nukleinsäuren in einer Peripherblutprobe, die von einer schwangeren Frau erhalten wurde.

2. Verfahren nach Anspruch 1, wobei das vorbestimmte Chromosom ein Autosom umfasst, wobei vorzugsweise das Autosom keines der Chromosomen 13, 18 und 21 umfasst.

3. Verfahren nach einem der Ansprüche 1 oder 2, wobei Schritt (2) ferner umfasst:

(2-1) Alignment des Sequenzierungsergebnisses an der Referenzgenomsequenz, um einen Datensatz zu konstruieren, der aus einer Vielzahl von eindeutig zugeordneten Sequenzierungs-Reads besteht, wobei jeder eindeutig zugeordnete Sequenzierungs-Read in dem Datensatz einer einzelnen Position in der Referenzgenomsequenz zugeordnet ist;
(2-2) Bestimmen der Position jedes eindeutig zugeordneten Sequenzierungs-Reads in der Referenzgenomsequenz; und
(2-3) Bestimmen der Anzahl der eindeutig zugeordneten Sequenzierungs-Reads, die in das vorbestimmte Fenster fallen.

4. Verfahren nach einem der Ansprüche 1 bis 3, wobei in Schritt (3):

Das neuronale Netzmodell ein TensorFlow-Lernsystem verwendet,
wobei vorzugsweise das TensorFlow-Lernsystem die folgenden Parameter umfasst:

die Anzahlen der eindeutig zugeordneten Sequenzierungs-Reads, die in einzelne Fenster eines Autosoms fallen, als eine Eingabeschicht;
einen fetalen Anteil als Ausgabeschicht;
ReLu als ein Neuron;
einen Optimierer, der aus mindestens einem der folgenden ausgewählt wird: Adam, SGD und Ftrl,
wobei vorzugsweise der Optimierer Ftrl ist,
wobei vorzugsweise das TensorFlow-Lernsystem ferner die folgenden Parameter umfasst:

eine Lernrate, die auf 0,002 eingestellt ist;
1 versteckte Schicht; und
200 Neuronen in der versteckten Schicht.

5. Verfahren nach Anspruch 4, wobei die Gewichtung jedes vorbestimmten Fensters durch ein statistisches Ridge-Regressionsmodell mit der folgenden Berechnungsformel bestimmt wird:

$$\hat{y} = \hat{\beta}_0 + \sum_{j=1}^{p} \hat{\beta}_j x_j \text{ ,}$$

wobei $\hat{y}$ ein prädiktiver fetaler Anteil ist, $x_j$ die Anzahl der eindeutig zugeordneten Sequenzierungs-Reads, die in ein Fenster fallen ist, $\hat{\beta}_j$ eine Gewichtung eines Fensters ist, $\hat{\beta}_0$ eine Abweichung ist, und $\hat{\beta}_j$ und $\hat{\beta}_0$ durch Training des Modells erhalten werden.

6. Verfahren nach Anspruch 4, wobei die Gewichtung jedes vorbestimmten Fensters durch ein neuronales Netzmodell mit der folgenden Berechnungsformel bestimmt wird:

$$z_j^l = f\left(\sum_k w_{jk}^l z_k^{l-1} + b_j^l\right) \text{ ,}$$

wobei $l$ eine fortlaufende Nummer einer Schicht in dem neuronalen Netzwerkmodell ist, zj ein Wert für ein $j$-tes Neuron in einer $l$-ten Schicht ist, $z_k^{l-1}$ ein Wert für ein k-tes Neuron in einer ($l$-1)-ten Schicht ist, $w_{jk}^l$ eine Verbindungsgewichtung vom k-ten Neuron in der ($l$-1)-ten Schicht zu dem $j$-ten Neuron in der $l$-ten Schicht ist,

$b_j^l$ eine Eingangsabweichung für das j-te Neuron in der *l*-ten Schicht ist, und w und b durch Training des Modells erhalten werden,

wobei vorzugsweise das Bestimmen der Gewichtung jedes vorbestimmten Fensters, das durch das neuronale Netzwerkmodell bestimmt wird, umfasst:

Berechnen eines Wertes für jedes Neuron Schicht für Schicht gemäß der Berechnungsformel des neuronalen Netzwerkmodells, wobei ein Wert für ein Neuron in der letzten Schicht der prädiktive fetale Anteil ist.

7. Verfahren nach einem der Ansprüche 1 bis 6, wobei vor Schritt (3) eine GC-Korrektur an der Anzahl der eindeutig zugeordneten Sequenzierungs-Reads, die in das vorbestimmte Fenster fallen, im Voraus durchgeführt wird, um die Anzahl der GC-korrigierten, eindeutig zugeordneten Sequenzierungs-Reads, die in das vorbestimmte Fenster fallen, zu erhalten,

wobei vorzugsweise die GC-Korrektur durchgeführt wird durch:

Abgleichen der Anzahl von eindeutig zugeordneten Sequenzierungs-Reads, die in einzelne vorbestimmte Fenster des vorbestimmten Chromosoms fallen, mit entsprechenden GC-Gehalten, um $ER = f(gc)$ zu bestimmen;
Durchführen einer Korrektur an der Anzahl der eindeutig zugeordneten Sequenzierungs-Reads für jedes vorbestimmte Fenster des vorbestimmten Chromosoms:

$$ERA_i = ER_i * (\overline{ER}/f(GC_i)), \quad i = 1,2,3,\dots,N ,$$

wobei für eine Probe $ER_i$ die Anzahl der eindeutig zugeordneten Sequenzierungs-Reads, die in ein i-tes vorbestimmtes Fenster fallen, darstellt, $GC_i$ einen GC-Gehalt einer Referenzsequenz für das i-te vorbestimmte Fenster darstellt, $\overline{ER}$ einen Mittelwert für die Anzahlen der eindeutig zugeordneten Sequenzierungs-Reads, die in einzelne vorbestimmte Fenster des vorbestimmten Chromosoms fallen, darstellt; und $ERA_i$ die Anzahl der GC-korrigierten, eindeutig zugeordneten Sequenzierungs-Reads in dem i-ten vorbestimmten Fenster nach der Korrektur darstellt.

8. Verfahren nach einem der Ansprüche 1 bis 7, wobei vor Schritt (3) das Geschlecht des Fötus bestimmt wird,
wobei vorzugsweise das Geschlecht des Fötus durch ein Verhältnis der Anzahl der eindeutig zugeordneten Sequenzierungs-Reads im Y-Chromosom zur Gesamtzahl der eindeutig zugeordneten Sequenzierungs-Reads in allen Chromosomen bestimmt wird.

## Revendications

1. Procédé de détermination d'une fraction d'acides nucléiques acellulaires d'une source prédéterminée dans un échantillon biologique, comprenant les étapes consistant à :

(1) effectuer le séquençage sur les acides nucléiques acellulaires contenus dans l'échantillon biologique, de manière à obtenir un résultat de séquençage consistant en une pluralité de lectures de séquençage ;
(2) aligner le résultat de séquençage avec une séquence de référence, de manière à déterminer le nombre de lectures de séquençage tombant dans une fenêtre prédéterminée dans le résultat de séquençage,

dans lequel la séquence de référence est une séquence génomique de référence,
dans lequel la fenêtre prédéterminée est obtenue par division séquentielle d'un chromosome prédéterminé de la séquence génomique de référence ; et

(3) déterminer la fraction des acides nucléiques acellulaires de la source prédéterminée dans l'échantillon biologique sur la base du nombre de lectures de séquençage tombant dans la fenêtre prédéterminée, dans lequel la fraction des acides nucléiques acellulaires de la source prédéterminée dans l'échantillon biologique est déterminée par un poids de chaque fenêtre prédéterminée,

dans lequel le poids de chaque fenêtre prédéterminée est prédéterminé avec des échantillons d'entraînement et est déterminé par au moins l'un d'un modèle statistique de régression des crêtes et d'un modèle de réseau de neurones,

dans lequel l'échantillon d'entraînement est un échantillon de sang périphérique avec une fraction connue d'acides nucléiques foetaux acellulaires d'une femme enceinte,

dans lequel l'échantillon d'entraînement est un échantillon de sang périphérique avec une fraction connue d'acides nucléiques foetaux acellulaires d'une femme enceinte avec un foetus mâle normal, où le foetus mâle normal se réfère à un foetus mâle avec des chromosomes normaux,

dans lequel l'échantillon biologique est un échantillon de sang périphérique,

dans lequel l'acide nucléique acellulaire de la source prédéterminée est au moins l'un sélectionné parmi les suivants :

des acides nucléiques foetaux acellulaires dans un échantillon de sang périphérique qui a été obtenu d'une femme enceinte ;

et

des acides nucléiques maternels acellulaires dans un échantillon de sang périphérique qui a été obtenu d'une femme enceinte.

2. Procédé selon la revendication 1, dans lequel le chromosome prédéterminé comprend un autosome : de préférence, dans lequel l'autosome ne comprend aucun des chromosomes 13, 18 et 21.

3. Procédé selon l'une quelconque des revendications 1 ou 2, dans lequel l'étape (2) comprend en outre :

(2-1) aligner le résultat du séquençage avec la séquence génomique de référence, de manière à construire un ensemble de données constitué d'une pluralité de lectures de séquençage mappées de manière unique, où chaque lecture de séquençage mappée de manière unique dans l'ensemble de données mappe une seule position dans la séquence génomique de référence ;

(2-2) déterminer la position de chaque lecture de séquençage mappée de manière unique dans la séquence génomique de référence ; et

(2-3) déterminer le nombre de lectures de séquençage mappées de manière unique tombant dans la fenêtre prédéterminée.

4. Procédé selon l'une quelconque des revendications 1 à 3, dans lequel à l'étape (3),

le modèle de réseau de neurones adopte un système d'apprentissage TesnsorFlow,

de préférence, dans lequel le système d'apprentissage TensorFlow comprend les paramètres suivants :

les nombres de lectures de séquençage mappées de manière unique tombant dans des fenêtres individuelles d'un autosome en tant que couche d'entrée ;

une fraction foetale en tant que couche de sortie ;

ReLu en tant que neurone ;

un optimiseur sélectionné parmi au moins l'un de Adam, SGD et Ftrl,

de préférence, dans lequel l'optimiseur est Ftrl,

de préférence, dans lequel le système d'apprentissage TensorFlow comprend en outre les paramètres suivants :

une vitesse d'apprentissage fixée à 0,002 ;

1 couche cachée ; et

200 neurones dans la couche cachée.

5. Procédé selon la revendication 4, dans lequel le poids de chaque fenêtre prédéterminée est déterminé par un modèle statistique de régression des crêtes ayant la formule de calcul suivante :

$$\hat{y} = \hat{\beta}_0 + \sum_{j=1}^{p} \hat{\beta}_j x_j,$$

où $\hat{y}$ est une fraction foetale prédictive, $x_j$ est le nombre de lectures de séquençage mappées de manière unique tombant dans une fenêtre, $\beta_j$ est le poids d'une fenêtre, $\beta_0$ est un écart, $\beta_j$ et $\beta_0$ sont obtenues en entraînant le modèle.

6. Procédé selon la revendication 4, dans lequel le poids de chaque fenêtre prédéterminée est déterminé par un modèle de réseau de neurones ayant la formule de calcul suivante :

$$z_j^l = f\left(\sum_k w_{jk}^l z_k^{l-1} + b_j^l\right)$$

,

où $l$ est un numéro de série d'une couche dans le modèle de réseau de neurones, $z_j^l$ est une valeur pour un $j^{\text{ème}}$ neurone dans une $l^{\text{ème}}$ couche, $z_k^{l-1}$ est une valeur pour un $k^{\text{ème}}$ neurone dans une $(l-1)^{\text{ème}}$ couche, $w_{jk}^l$ un poids de connexion du $k^{\text{ème}}$ neurone dans la $(l-1)^{\text{ème}}$ couche au $j^{\text{ème}}$ neurone dans la $l^{\text{ème}}$ couche, $b_j^l$ est un écart d'entrée pour le $j^{\text{ème}}$ neurone dans la $l^{\text{ème}}$ couche, et w et b sont obtenus en entraînant le modèle, de préférence, dans lequel la détermination du poids de chaque fenêtre prédéterminée déterminée par le modèle de réseau de neurones 15 comprend :

calculer une valeur pour chaque neurone couche par couche selon la formule de calcul du modèle de réseau de neurones, où une valeur pour un neurone dans la dernière couche est la fraction foetale prédictive.

**7.** Procédé selon l'une quelconque des revendications 1 à 6, dans lequel avant l'étape (3), une correction en GC est effectuée sur le nombre de lectures de séquençage mappées de manière unique tombant dans la fenêtre prédéterminée à l'avance, de manière à obtenir le nombre de lectures de séquençage mappées de manière unique corrigées en GC tombant dans la fenêtre prédéterminée,
de préférence, dans lequel la correction en GC est effectuée par :

la correspondance des nombres de lectures de séquençage mappées de manière unique tombant dans des fenêtres prédéterminées individuelles du chromosome prédéterminé avec des teneurs en GC correspondantes pour déterminer ER = f(gc) ;
l'exécution d'une correction sur le nombre de lectures de séquençage mappées de manière unique pour chaque fenêtre prédéterminée du chromosome prédéterminé :

$$ERA_i = ER_i * (\overline{ER}/f(GC_i)), \quad i = 1,2,3,\dots,N$$

où pour un échantillon, $ER_i$ représente le nombre de 5 lectures de séquençage mappées de manière unique tombant dans une $i^{\text{ème}}$ fenêtre prédéterminée, $ER_{A_i}$ $GC_i$ représente une teneur en GC d'une séquence de référence pour la $i^{\text{ème}}$ fenêtre prédéterminée, $\overline{ER}$ représente une valeur moyenne pour les nombres de lectures de séquençage mappées de manière unique tombant dans des fenêtres prédéterminées individuelles du chromosome prédéterminé ; et représente le nombre de lectures de séquençage mappées de manière unique corrigées en GC dans la $i^{\text{ème}}$ fenêtre prédéterminée après correction.

**8.** Procédé selon l'une quelconque des revendications 1 à 7, dans lequel, avant l'étape (3), le sexe du foetus est prédéterminé,
de préférence, dans lequel le sexe du foetus est déterminé par un rapport entre le nombre de lectures de séquençage mappées de manière unique dans le chromosome Y et le nombre total de 15 lectures de séquençage mappées de manière unique dans tous les chromosomes.

S100

nucleic acid sequencing

S200

determining the number of
sequencing reads falling into a
predetermined window

S300

determining the fraction of
cell-free nucleic acids

Fig. 1

S210

the sequencing result is aligned to
the reference genome sequence

S220

the position of each uniquely-
mapped sequencing read in the
reference genome sequence is
determined

S230

the number of the sequencing reads
falling into the predetermined
window is determined

Fig. 2

```
┌─────────────────────────────┐
│                             │      100
│     sequencing apparatus    │
│                             │
└─────────────────────────────┘
                │
                │
┌─────────────────────────────┐
│                             │      200
│     counting apparatus      │
│                             │
└─────────────────────────────┘
                │
                │
┌─────────────────────────────┐
│   fraction of cell-free     │      300
│   nucleic acids             │
│   determining apparatus     │
└─────────────────────────────┘
```

Fig. 3

```
┌─────────────────────────────┐
│                             │      210
│       aligning unit         │
│                             │
└─────────────────────────────┘
                │
                │
┌─────────────────────────────┐
│    position determining     │      220
│           unit              │
│                             │
└─────────────────────────────┘
                │
                │
┌─────────────────────────────┐
│    number determining       │      230
│           unit              │
│                             │
└─────────────────────────────┘
```

Fig. 4

Fig. 5

Fig. 6

Fig. 7

Fig. 8

Fig. 9